# EUROPÄISCHE PATENTSCHRIFT

(11) **EP 0 269 791 B1**
(45) Veröffentlichungstag und Bekanntmachung des Hinweises auf die Patenterteilung: **03.03.1993**
(21) Anmeldenummer: 87111674.5
(22) Anmeldetag: 12.08.1987
(51) Int. Cl.: A61B 8/12, A61B 1/00, A61B 17/36

(54) **Endoskop mit einem Ultraschallwandler**
Endoscope with an ultrasonic transducer
Endoscope à transducteur d'ultrasons

(30) Priorität: 22.11.1986 DE 3639981
(43) Veröffentlichungstag der Anmeldung: 08.06.1988
(73) Patentinhaber: Deutsche Aerospace AG, 81663 München (DE)
(72) Erfinder: Frank, Frank, Dr., D-8017 Ebersberg (DE); Hahn, Andreas, D-8029 Sauerlach (DE); Pensel, Jürgen, Dr., D-2401 Grossgrönau (DE); Rothenberger, Karl-Heinz, Dr., D-8300 Landshut (DE)

(56) Entgegenhaltungen:
- EP-A- 0 046 987
- WO-A-85/00010
- WO-A-85/00510
- US-A- 4 582 067
- US-A- 4 587 972

## Beschreibung

Die Erfindung bezieht sich auf ein Endoskop, insbesondere zur Untersuchung und Behandlung von Tumoren, etwa Blasentumoren, nach dem Oberbegriff des Patentanspruchs 1.

Bei einem bekannten Endoskop dieser Art (EP-A-46 987) zur visuellen Inspektion von Körperhöhlen ist zusätzlich zur Sichtoptik am distalen Endoskopende ein die Sichtoptik ringförmig umschließender Ultraschallwandler vorgesehen, mit dessen Hilfe der Abstand des distalen Endoskopendes von der inspizierten Gewebeoberfläche gemessen und hieraus der Größenmaßstab der okularseitigen Abbildung ermittelt werden kann.

Ferner ist aus der US-A-4 587 972 ein Ultraschallkatheter mit einem in den Ultraschallwandler eingebetteten Laserlichtbündel zur Beseitigung von Cefäßobstruktionen bekannt, bei dem die Laserbestrahlung und die Ultraschallmessung in zeitlichem Wechsel zueinander erfolgen derart, daß die Laserbehandlung jeweils für die Dauer einer Ultraschall-Kontrollaufnahme unterbrochen wird, um so die Größe der noch zu entfernenden Obstruktionsreste festzustellen.

Aufgabe der Erfindung ist es, ein Endoskop der eingangs genannten Art so weiterzubilden, daß die zur endoskopischen Laserbestrahlung erforderlichen Untersuchungs- und Behandlungsschritte gleichzeitig durchführbar sind.

Diese Aufgabe wird erfindungsgemäß durch das im Patentanspruch 1 gekennzeichnete Endoskop gelöst.

Mit dem erfindungsgemäßen Endoskop läßt sich simultan zur Laserbehandlung sowohl eine Ultraschall-Tiefenmessung als auch eine hierzu genau ortskorrellierte visuelle Oberflächeninspektion erzielen und mit Hilfe der Steuereinheit die Laserbestrahlung für die Koagulation in Echtzeit und automatisch nach Maßgabe der durch die Ultraschallmessung ermittelten Koagulationstiefe steuern. Hierdurch wird eine sehr genaue, rasche und für den Patienten schonende Tumorentfernung ermöglicht.

In weiterer vorteilhafter Ausgestaltung der Erfindung wird nach Anspruch 2 mit Hilfe des Ultraschallwandlers zusätzlich auch der Abstand des distalen Endoskopendes von dem zu bestrahlenden Bereich ermittelt und die Laserleistung mittels der Steuereinheit den Abstandsvariationen nachgesteuert, wodurch auf einfache Weise aufgrund der divergenten Abstrahlchrakteristik abstandsabhängige Änderungen der Laserleistungsdichte an dem zu behandelnden Bereich kompensiert werden.

Vorzugsweise wird die Laserhestrahlung gemäß Anspruch 3 aus Sicherheitsgründen durch die Steuereinheit bei Erreichen einer vorgegebenen, durch die Ultraschallmessung ermittelten Koagulationstiefe asgebrochen.

Die im Anspruch 4 gekennzeichnete Ausbildung erlaubt eine genaue Beobachtung des Behandlungserfolges ohne Parallaxenfehler. Durch die Anordnung gemäß Anspruch 5 bzw. 6 wird das verfügbare Lumen optimal ausgenutzt.
Weitere Mittel, beispielsweise Spülleitungen und Beleuchtungslichtleiter sind vorzugsweise gemäß Anspruch 7 ebenfalls innerhalb der Abstrahlfläche des Ultraschallwandlers angeordnet.

Die Erfindung wird nachstehend anband von Ausführungsbeispielen unter Bezugnahme auf die Zeichnung näher beschrieben, in der zeigen:
- Fig. 1:: eine Seitenansicht eines erfindungsgemäß ausgebildeten Endoskops,
- Fig. 2:: einen Schnitt längs der Linie II-II der Fig. 1 und
- Fig. 3:: einen Schritt längs der Linie III-III der Fig. 1.

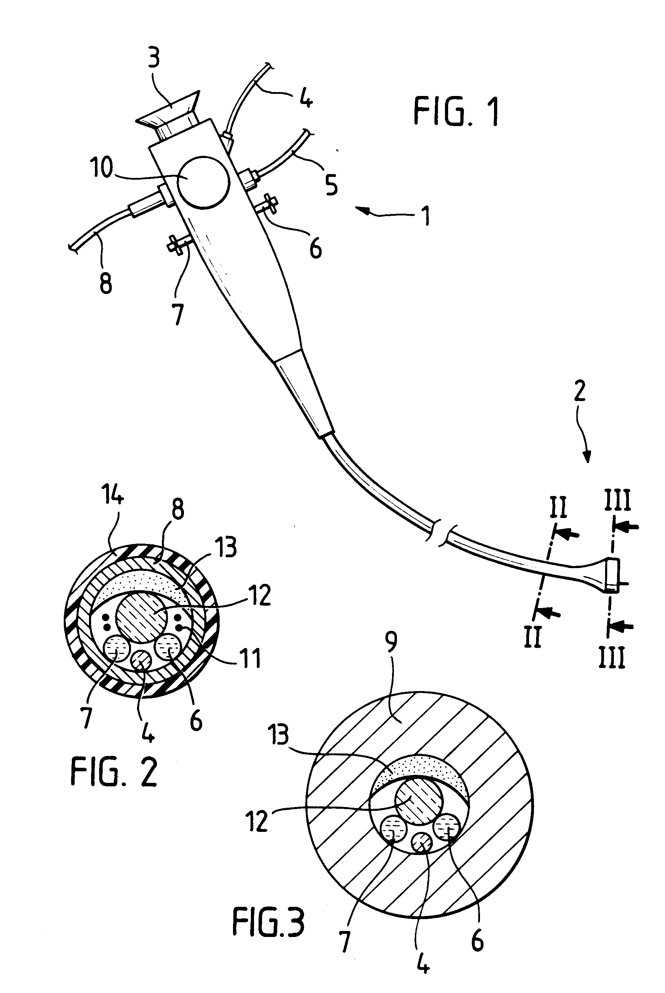

Das in den Figuren dargestellte erfindungsgemäße Endoskop weist in an sich bekannter Weise ein Handstück 1 und einen eigentlichen Endoskop-Teil 2 auf. An dem Handstück sind ein Okkular 3 sowie verschiedene Leitungen angebracht. Bei dem gezeigten Ausführungsbeispiel sind dies: ein Laserlichtleiter 4, ein Kaltlichtleiter 5 für das Beleuchtungslicht, ein Spülmittel-Zufluß 6, ein Spülmittel-Abfluß 7 und ein Transducersignalkabel für einen Ultraschallwandler 9 (Fig. 3). Ferner sind in an sich bekannter Weise Stellknöpfe 10 für Seilzüge 11 vorgesehen, die zum Abwinkeln des Endoskops dienen.

Die Fig. 2 und 3 zeigen Schritte durch den eigentlichen Endoskop-Teil 2 längs der Linie II-II bzw. III-III in Fig. 1. Wie den Figuren zu entnehmen ist, ist eine Sichtoptik 12 zentral in dem Endoskop-Teil 2 angeordnet. Konzentrisch zu der Sichtoptik 12 ist nach Art eines Koaxialkabels das Transdusersignalkabel 8 (Fig. 2) bzw. der Ultraschalltransduser (Ultraschallwandler) 9 (Fig. 3) angeordnet. Zwischen dem Signalkabel 8 und der Sichtoptik 12 befinden sich der Laserlichtleiter 4, die Spülmittel-Zu- bzw. -Abflüsse 6 und 7, die Seilzüge 11 und ein Kaltlichtfaserbündel 13, so daß das verfügbare Lumen optimal ausgenutzt ist. Das Signalkabel 8 wird von einem Einbettungsmaterial 14 für den Transduser 9 umgeben, durch das der Wirkungsgrad erhöht wird.

Vorstehend ist die Erfindung anhand eines Ausführungsbeispiels ohne Beschränkung des allgemeinen Erfindungsgedankens beschrieben worden. Im Rahmen des Erfindungsgedankens sind selbstverständlich die verschiedensten Modifikationen möglich:
So kann der erfindungsgemäße Grundgedanke auch bei einem starrem Endoskop verwirklicht werden.

Die Anordnung der verschiedenen Leitungen kann selbstverständlich ebenfalls von der dargestellten abweichen. Ferner können auch zusätzliche Leitungen vorgesehen werden.

Die Steuereinheit, die u.a. die Laserleistung in Abhängigkeit von den Ausgangssignalen des Ultraschallwandlers steuert, kann beispielsweise mit einem Mikrocomputer aufgebaut sein. Deshalb muß der Aufbau nicht näher erläutert werden.

Darüberninaus kann auch eine Bildverarbeitungseinrichtung angeschlossen werden, die das Bild der Sichtoptik verarbeitet und deren Signale ebenfalls an die Steuereinheit angelegt sein können.

## Patentansprüche

1. Endoskop, insbesondere zur Untersuchung und Behandlung von Tumoren, beispielsweise Blasentumoren, mit einer Sichtoptik und einem am distalen Endoskopende angeordneten Ultraschallwandler, dessen Abstrahlfläche ringförmig ausgebildet ist und das distale Ende der Sichtoptik umgibt,
dadurch **gekennzeichnet,** daß
innerhalb der ringförmigen Abstrahlfläche des Ultraschallwandlers (9) zusätzlich zur Sichtoptik (12) das Austrittsende eines Laserlichtleiters (4) für einen Laser-Behandlungsstrahl angeordnet ist und daß auf Basis der Messungen des Ultraschallwandlers die Tiefenausdehnung eines Tumors und die erreichte Koagulationstiefe simultan zur Laserbestrahlung ermittelt werden können und eine gleichzeitig mit der Ultraschallmessung wirksame Steuereinheit vorgesehen ist, die die Laserbehandlung nach Maßgabe der ermittelten Koagulationstiefe steuert.

2. Endoskop nach Anspruch 1,
dadurch **gekennzeichnet,** daß
der Ultraschallwandler (9) zusätzlich den Abstand des distalen Endoskopendes von dem zu bestrahlenden Bereich ermittelt und die Steuereinheit die Laserleistung entsprechend dem ermittelten Abstand steuert.

3. Endoskop nach Anspruch 1 oder 2,
dadurch **gekennzeichnet,** daß
die Steuereinheit die Laserbestrahlung bei Erreichen einer bestimmten Koagulationstiefe beendet.

4. Endoskop nach einem der vorhergehenden Ansprüche,
dadurch **gekennzeichnet,** daß
die Sichtoptik (12) konzentrisch zur Abstrahlfläche des Ultraschallwandlers (9) angeordnet ist.

5. Endoskop nach einem der vorhergehenden Ansprüche,
dadurch **gekennzeichnet,** daß
das Signalkabel (8) für den Ultraschallwandler (9) den Laserlichtleiter (4) und die Sichtoptik (12) ringförmig umschließt.

6. Endoskop nach einem der vorhergehenden Ansprüche,
dadurch **gekennzeichnet,** daß
die Sichtoptik (12) in an sich bekannter Weise flexibel ausgebildet ist und daß Zugseile (11) für das Abwinkeln der Sichtoptik innerhalb der Ultraschall-Abstrahlfläche sowie ggf. innerhalb des Ultraschall-Signalkabels (8) verlaufen.

7. Endoskop nach einem der vorhergehenden Ansprüche,
dadurch **gekennzeichnet,** daß
Spülleitungen (6, 7) und Beleuchtungslichtleiter (13) ebenfalls innerhalb der Ultraschall-Abstrahlfläche sowie ggf. innerhalb des Ultraschall-Signalkabels (8) verlaufen.

## Claims

1. Endoscope, in particular for examination and treatment of tumours, for example bladder tumours, comprising sight optics and at the distal end of the endoscope an ultra-sound converter, the radiating surface of which is annular and embraces the distal end of the sight optics, **characterised in that** within the annular radiation surface of the ultra-sound converter (9) and in addition to the sight optics (12) is arranged the output end of a laser-light conductor (4) for a laser-treatment beam, and that on the basis of measurements taken by the ultra-sound converter (3) the depth of a tumour and an achieved coagulation depth can be determined simultaneously for laser treatment, and that a control unit is provided which simultaneously co-acts with the ultra-sound measuring and which controls the laser treatment according to a determined coagulation depth.

2. Endoscope according to claim 1, **characterised in that** the ultra-sound converter (9) additionally determines the distance of the endoscope's distal end from an area to be radiated and that the control unit controls the laser output according to the determined distance.

3. Endoscope according to claim 1 or 2, **characterised in that** the control unit terminates laser radiation on arriving at a specified coagulation depth.

4. Endoscope according to one of the above claims, **characterised in that** the sight optics (12) are arranged concentrically to the radiation surface of the ultra-sound converter (9).

5. Endoscope according to one of the above claims, **characterised in that** a signal cable (8) for the ultra-Round converter (9) annularly encloses the laser-light conductor (4) and the sight optics (12).

6. Endoscope according to one of the above claims, **characterised in that** the sight optics (12) are flexibly arranged in a conventional manner and the pull wires (11) for angular positioning of the sight optics extend within the ultra-sound radiation surface and, if appropriate, within the ultra-sound signal cable (8).

7. Endoscope according to one of the above claims, **characterised in that** flushing pipes (6, 7) and illumination conductors (13) also extend within the ultra-sound radiation surface and, if appropriate, within the ultra-sound signal cable (8).

## Revendications

1. Endoscope, en particulier pour l'examen et le traitement de tumeurs, par exemple de tumeurs de la vessie, comportant une optique de vision et un transducteur à ultrasons qui est disposé à l'extrémité distale de l'endoscope et dont la surface émettrice est annulaire et entoure l'extrémité distale de l'optique de vision, caractérisé par le fait que l'extrémité de sortie d'une fibre optique de laser (4) conduisant un faisceau laser de traitement est disposée avec l'optique de vision (12) à l'intérieur de la surface émettrice annulaire du transducteur à ultrasons (9) et par le fait que, sur la base des mesures du transducteur à ultrasons, on détermine l'étendue en profondeur d'une tumeur et la profondeur de coagulation obtenue simultanément à l'irradiation laser et qu'il est prévu une unité de commande qui agit simultanément avec la mesure à ultrasons, laquelle unité commande le traitement laser en fonction de la profondeur de coagulation déterminée.

2. Endoscope selon la revendication 1, caractérisé par le fait que le transducteur à ultrasons (9) détermine en outre la distance de l'extrémité distale de l'endoscope par rapport à la zone à irradier et l'unité de commande pilote la puissance du laser en fonction de la distance déterminée.

3. Endoscope selon la revendication 1 ou 2, caractérisé par le fait que l'unité de commande interrompt l'irradiation laser dès qu'une profondeur de coagulation déterminée est atteinte.

4. Endoscope selon l'une des revendications précédentes, caractérisé par le fait que l'optique de vision (12) est concentrique avec la surface émettrice du transducteur à ultrasons (9).

5. Endoscope selon l'une des revendications précédentes, caractérisé par le fait que le câble de signal (8) menant au transducteur à ultrasons entoure la fibre optique (4) et l'optique de vision (12) à la manière d'un anneau.

6. Endoscope selon l'une des revendications précédentes, caractérisé par le fait que, de manière connue en soi, l'optique de vision (12) est souple et que des câbles de traction (11) permettant de faire pivoter l'optique de vision s'étendent dans la surface émettrice d'ultrasons et éventuellement à l'intérieur du câble de signal (8).

7. Endoscope selon l'une des revendications précédentes, caractérisé par le fait que des conduits de lavage (6, 7) et des fibres optiques d'éclairage (13) s'étendent également dans la surface émettrice d'ultrasons et éventuellement à l'intérieur du câble de signal (8).
